# EUROPÄISCHE PATENTSCHRIFT

(11) **EP 0 752 408 B1**
(45) Veröffentlichungstag und Bekanntmachung des Hinweises auf die Patenterteilung: **10.01.2001**
(21) Anmeldenummer: 96110449.4
(22) Anmeldetag: 28.06.1996
(51) Int. Cl.: C07C 69/96

(54) **Strahlungshärtbare Acrylate mit eingebauten Photoinitiatoren**
Radiation curable acrylates incorporating photoinitiators
Acrylates durcissables par radiation avec des photoimitiateurs incorporés

(30) Priorität: 07.07.1995 DE 19524812
(43) Veröffentlichungstag der Anmeldung: 08.01.1997
(73) Patentinhaber: BASF AKTIENGESELLSCHAFT, 67056 Ludwigshafen (DE)
(72) Erfinder: Reich, Wolfgang, Dr., 67133 Maxdorf (DE); Beck, Erich, Dr., 68528 Ladenburg (DE); Jäger, Ulrich, Dr., 67376 Harthausen (DE); Schwalm, Reinhold, Dr., 67157 Wachenheim (DE)

(56) Entgegenhaltungen:
- EP-A- 0 377 191

## Beschreibung

Die Erfindung betrifft strahlungshärtbare (Meth)acrylate, erhältlich durch Umsetzung von Verbindungen der Formel 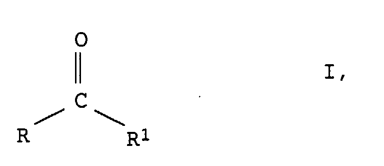 wobei R für einen C₁-C₄-Alkylrest, einen Arylrest oder den Rest R¹ steht und
R¹ für den Rest 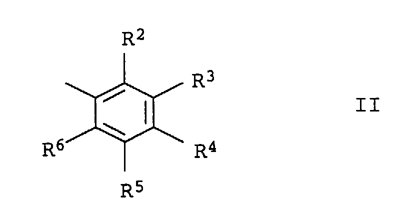 steht, wobei die Reste R² bis R⁶ unabhängig voneinander für H, C₁-C₄-Alkyl, C₁-C₄-Alkoxy, OH, Phenyl, SH, SCH₃, SC₂H₅, F, Cl, Br, CN, COOH, COO-(C₁-C₁₇-Alkyl), COO-C₅-C₁₀-Aryl), CF₃, N(Alkyl)₂, N((Alkyl)(Aryl), N(Aryl)₂, N^{⊕}(Alkyl)₃ A^{⊖}, N^{⊕}(Alkyl)₂A^{⊖} stehen, A^{⊖} das Anion einer Säure und Alkyl-, bzw. Aryl, soweit nichts anderes angegeben, eine C₁-C₁₀-Alkyl bzw. eine C₅-C₁₀-Arylgruppe bedeutet und mindestens einer, aber maximal 3 der Reste R² bis R⁶ eine Gruppe 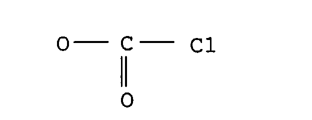 sind, mit Hydroxy(meth)acrylaten, welche mindestens 1 freie Hydroxygruppe und mindestens 2 (Meth)acrylgruppen, im Molekül enthalten, bei 0 bis 100°C unter Feuchtigkeitsausschuß. Weiterhin betrifft die Erfindung ein Verfahren zur Herstellung der strahlungshärtbaren (Meth)acrylate sowie ihre Verwendung in strahlungshärtbaren Massen.

Es ist bekannt, copolymerisierbare Photoinitiatoren als Comonomere in Polymere einzubauen. Eine spätere Migration oder Verflüchtigung der Photoinitiatoren aus hergestellten Überzügen kann so weitgehend vermieden werden.

Copolymerisierbare bzw. coreaktive Photoinitiatoren sind auch aus. der EP 281 941 bekannt.

Aus der EP-A-377 191 sind copolymerisierbare Photoinitiatoren bekannt, welche durch Umsetzung von Verbindungen der obigen Formel I mit Monoacrylaten erhältlich sind.

Nachteilig ist, daß Monoacrylate bei der Strahlungshärtung oft nur unvollständig in das Polymer eingebaut werden und es so zu einem unerwünschten Entweichen der Photoinitiatoren aus hergestellten Überzügen kommen kann.

Aufgabe der vorliegenden Erfindung waren daher strahlungshärtbare (Meth)acrylate hoher Reaktivität mit geringen Anteilen extrahierbarer Photoinitiatoren.

Demgemäß wurden die oben definierten strahlungshärtbaren (Meth)acrylate, ein Verfahren zu ihrer Herstellung, sowie ihre Verwendung in strahlungshärtbaren Massen gefunden.

Die erfindungsgemäßen, strahlungshärtbaren (Meth)acrylate sind erhältlich durch Umsetzung von Verbindungen der Formel I mit Hydroxy(meth)acrylaten, welche mindestens eine freie Hydroxylgruppe und mindestens 2 (Meth)acrylgruppen enthalten.

In Formel I steht R bevorzugt für einen Methylrest und besonders bevorzugt für einen Phenylrest. Die Reste R² bis R⁶ stehen bevorzugt unabhängig voneinander für ein H-Atom, eine C₁-C₄-Alkyl- oder Alkoxygruppe oder für eine nicht in ortho-Position zur Carbonylgruppe stehende OH-Gruppe, wobei ein bis drei der Reste R² bis R⁶, vorzugsweise einer der Reste R² bis R⁶, für eine Gruppe 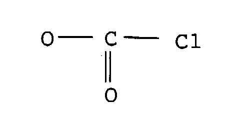 steht. Eine besonders bevorzugte Verbindung der Formel I ist

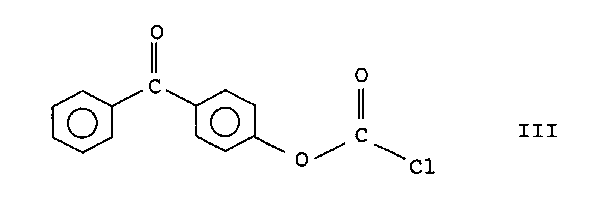

Die aromatische Chlorformiate (vgl. J. Prakt. Chem. 313, 331 (1971), dito 317, 62, 73, 81 (1975)) der allgemeinen Formel I lassen sich aus einem substituierten Phenol, z.B. 4-Chlor-5'-fluor-2'-hydroxybenzophenon, 4-Chlor-4'hydroxybenzophenon, 2,4-Dihydroxybenzophenon, 4,4'-Dihydroxybenzophenon, 4-Fluor-4, -hydroxybenzophenon, 2-Hydroxybenzophenon, 4-Hydroxybenzophenon, 2-Hydroxy-4-methoxybenzophenon, 2,3,4-Trihydroxybenzophenon, 2-Hydroxy-thioxanthon, 3-Hydroxy-thioxanthon, (4-Hydroxyphenyl)-2-hydroxy-2-propylketon (DE-OS 35 34 645) durch Phosgenierung nach literaturbekannten Standardverfahren mit Phosgen, s. z.B. Houben-Weyl, Methoden der organischen Chemie, Bd. 8, Thieme-Verlag 1952, Trichlormethylchlorformiat (Diphosgen), J. Prakt. Chem. 126, 210 (1930), dito 128, 233 (1930), Chem.-Abstr. 95, 81766, J. Org. Chem. 50, 715 (1985), J. Org.Chem. 41, 2070 (1976), Angew. Chem. 89, 267 (1977), dem kristallinen Triphosgen, Angew. Chem. 99, 922 (1987), N,N'-Carbonyldiimidazol oder N,N'-Carbonyldi-s-triazol (Fieser 1, 116 (1967)), in guten Ausbeuten herstellen.

Die Hydroxy(meth)acrylate, welche mit der Verbindung I zu den erfindungsgemäßen strahlungshärtbaren (Meth)acrylaten umgesetzt werden können, enthalten mindestens eine, vorzugsweise ein bis 4 besonders bevorzugt, 1-2 Hydroxylgruppen. Die Hydroxylgruppen können sich dabei im mit der (Meth)acrylsäure veresterten Alkohol befinden oder aber z.B. auch durch Michael-Addition von primären oder sekundären Aminen an (Meth)acrylgruppen, insbesondere an Acrylgruppen, welche durch z.B. durch eine Hydroxyalkylgruppe substituiert sind, in das Molekül eingeführt sein.

Des weiteren enthalten sie mindestens 2, vorzugsweise 2 bis 6, besonders bevorzugt 2 bis 4 Acrylgruppen bzw. Methacrylgruppen im Molekül. Bevorzugt handelt es sich um Acrylgruppen und entsprechend um Hydroxyacrylate.

Geeignete Hydroxy(meth)acrylate sind z.B. Trimethylolpropandiacrylat und Pentaerythrittriacrylat.

Genannt seien auch Epoxidacrylate, wie sie z.B. durch Umsetzung von epoxidierten Olefinen, Glycidylestern von gesättigten oder ungesättigten Carbonsäuren oder Glycidylethern aliphatischer oder aromatischer Polyole mit (Meth)acrylsäure erhältlich sind.

Des weiteren kommen auch Urethanacrylate in Betracht, welche z.B. durch Umsetzung von Polyisocyanaten mit Hydroxylgruppen enthaltenden (Meth)acrylsäurestern hergestellt werden können.

Bevorzugte Hydroxy(meth)acrylate sind Polyester- oder Polyether(meth)acrylate mit mindestens einer freien Hydroxylgruppe und 2 bis 6, bevorzugt 2-4 Acrylgruppen im Molekül.

Die Polyester- bzw. Polyether(meth)acrylate können durch die dem Fachmann bekannte Veresterung von Hydroxylgruppen enthaltenden Polyestern oder Polyethern mit (Meth)acrylsäure hergestellt werden, wobei (Meth)acrylsäure in solchen Mengen eingesetzt wird, daß die gewünschte Anzahl von freien Hydroxylgruppen im Molekül verbleibt.

Die Molekulargewichte Mₙ der Hydroxylgruppen enthaltenden Polyester bzw. Polyether liegen bevorzugt zwischen 100 und 4000 (Mₙ bestimmt durch Gelpermeationschromatographie).

Derartige hydroxylgruppenhaltige Polyester können z.B. in üblicher Weise durch Veresterung von Dicarbonsäuren oder Polycarbonsäuren mit Polyolen mit mindestens 3 OH-Gruppen, gegebenenfalls im Gemisch mit Diolen, hergestellt werden. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride, z.B. Maleinsäureanhydrid oder Dialkylester der genannten Säuren eingesetzt werden. Als Polycarbonsäure, bzw. deren Anhydride seien Tri- oder Tetrasäuren wie Trimellitsäureanhydrid oder Benzoltetracarbonsäure genannt. Als Diole kommen vorzugsweise in Betracht Ethylenglykol, Propylenglykol-1,2 und -1,3, Butandiol-1,4, Hexandiol-1,6, Neopentylglykol, Cyclohexandimethanol sowie Polyglykole vom Typ des Ethylenglykols und Propylenglykols. Als Polyole sind in erster Linie Trimethylolpropan, Glycerin oder Pentaerythrit oder deren Dimere, z.B. Sorbit, zu nennen. Als Diole oder Polyole in Betracht kommen auch oxalkylierte (z.B. mit Ethylenoxid oder Propylenoxid) Diole oder Polyole, insbesondere mit einem Oxalkylierungsgrad von 0 bis 10, bezogen auf die jeweiligen Hydroxygruppen des Diols oder Polyols.

Zu den Polyesterolen zählen auch Polycaprolactontriole, deren Herstellung dem Fachmann ebenfalls bekannt ist.

Als hydroxylgruppenhaltige Polyether kommen z.B. solche in Frage, welche nach bekannten Verfahren durch Umsetzung von mehrwertigen Alkoholen mit verschiedenen Mengen an Alkylenoxiden, bevorzugt Ethylenoxid und/oder Propylenoxid erhalten werden können.

Bevorzugt sind Oxalkylierungsprodukte der obengenannten Polyole, insbesondere mit einem Oxalkylierungsgrad von 0 bis 10, bezogen auf die jeweiligen Hydroxylgruppen des Polyols, wobei jedoch insgesamt mindestens 2 Ethergruppen im Molekül vorhanden sind.

Bei der Herstellung der erfindungsgemäßen, strahlungshärtbaren (Meth)acrylate arbeitet man unter Feuchtigkeitsausschluß. Als Lösungsmittel eignen sich z.B. getrocknete, nicht nucleophile Lösungsmittel, wie Acetonitril, Dichlormethan, Dichlorethan, Tetrahydrofuran, Toluol, Xylol, Chlorbenzol, Essigester oder Chloroform.

Die Umsetzung der Hydroxy(meth)acrylate mit den Chlorformiaten der Formel I kann bei 0 bis 100, vorzugsweise 10 bis 50°C erfolgen. Falls die (Meth)acrylate flüssig sind, kann auf Lösungsmittel ganz oder zum Teil verzichtet werden.

Die erfindungsgemäßen, strahlungshärtbaren (Meth)-Acrylate können allein oder auch im Gemisch mit anderen, strahlungshärtbaren Polymeren oder Monomeren als strahlungshärtbare Massen verwendet werden.

Bei Gemischen sollte der Anteil der erfindungsgemäßen Acrylate im allgemeinen mindestens 0,5 vorzugsweise mindestens 2 Gew.-%, bezogen auf das Gemisch betragen, um eine ausreichende Reaktivität für die Strahlungshärtung ohne Zusatz weiterer Photoinitiatoren zu gewährleisten.

Gute Ergebnisse werden insbesondere mit einem Gehalt von 2 bis 40, insbesondere bis 20 Gew.-% der erfindungsgemäßen strahlungshärtbaren Acrylate, bezogen auf das strahlungshärtbare Gemisch erzielt. Als Mischungskomponente eignen sich insbesondere strahlungshärtbare (Meth)acrylate wie Epoxid(meth)acrylate, Urethan(meth)acrylate, Polyether- oder Polyester(meth)acrylate oder C₁-C₁₈-Alkyl(meth)acrylate und C₂-C₁₈-Alkylendi(meth)acrylate, aber auch Monomere wie Alkylacrylester, Vinylaromaten, Vinylester, unges. Polyester etc.

Bei den erfindungsgemäßen Acrylaten mit den voranstehend genannten Mischungskomponenten können ein Teil der (Meth)-Acrylgruppen, z.B. 0 bis 30 mol-%, vorzugsweise 0,1 bis 30 mol-%, besonders bevorzugt 0,5 bis 10 mol-%, bezogen auf alle (Meth)-Acrylgruppen in Form von Michael-Addukten von primären oder sekundären Aminen an die Acrylgruppe vorliegen, wodurch die Reaktivität bei der Strahlungshärtung erhöht werden kann.

Die strahlungshärtbaren Massen können als bzw. in Beschichtungsmassen, z.B. Lacken, Druckfarben, oder Klebstoffen, als Druckplatten, als Formkörper, zur Herstellung von Photoresisten, in der Stereolithographie oder als Gießmasse, z.B. für optische Linsen verwendet werden.

Zur Verwendung als Überzugsmasse können den erfindungsgemäßen strahlungshärtbaren Acrylaten bzw. den genannten Gemischen z.B. Zusatzstoffe wie Vernetzer, Verdicker, Verlaufsmittel, Füllstoffe oder Pigmente zugesetzt werden.

Die Strahlungshärtung kann durch Bestrahlen mit UV-Licht erfolgen.

### Beispiele

1. 100 g eines Polyetheracrylates (Laromer® LR 8812) wurden mit 5,75 g Chlorformiat der Formel III und 3,3 g Triethanolamin bei Raumtemperatur versetzt. Anschließend wurden 300 ml Essigester zugegeben. Nach 2 Stunden wurde entstandenes Triethanolammoniumchlorid mit destilliertem Wasser im Scheidetrichter ausgewaschen. Nach Abtrennung der wäßrigen Phase wurde der Essigester abdestilliert. (Viskosität des Endproduktes: 928 mPas).
2. 100 g Butandioldiglycidetherdiacrylat wurden mit 5,75 g Chlorformiat der Formel III und 3,3 g Triethanolamin 2 Stunden bei 50°C zur Reaktion gebracht. Anschließend wurde entstandenes Triethanolammoniumchlorid über einen Druckfilter abgetrennt. (Viskosität: 880 mPas).
3. Die Versuchsdurchführung entsprach Beispiel 2). Es wurden jedoch 100 g Bisphenol-A-diglycidetherdiacrylat (80%ig in Butylacetat) mit 5,75 g Chlorformiat der Formel III und 3,3 g Triethanolamin zur Reaktion gebracht.
4. 100 g Butandiolglycidetherdiacrylat wurden bei 70° mit 5 g Diethanolamin versetzt. Nach 4 Stunden Reaktion wurde auf 50°C abgekühlt und 5,75 g Chlorformiat der Formel III und 3,3 g Triethanolamin zugegeben. Nach 2 Stunden wurde das Ammoniumsalz über einen Druckfilter abgetrennt.
5. 375 g eines ethoxylierten Trimethylolpropantriacrylats wurden bei 70°C mit 46 g Diethanolamin versetzt. Nach 3 Stunden Reaktionsdauer wurde filtriert und abgefüllt. (Viskosität: 320 mPas)
   100 g dieses Produktes wurden mit 10 g Chlorformiat III, 100 g Essigester und 3,3 g Triethanolamin versetzt und auf 50°C aufgewärmt. Nach 4 h Reaktionsdauer wurde über einen K7-Druckfilter das Ammoniumsalz abgetrennt und anschließend der Essigester abdestilliert. (Produkt: Säurezahl (SZ): 6,0 mg KOH/g; Jodfarbzahl (JFZ): 7 - 10; Viskosität: 660 mPas).
6. 1000 g eines Polyetheracrylats (Laromer PO 33 F) wurden mit 100 g Chlorformiat der Formel III und 57,3 g Triethanolamin bei 55°C 2,5 Stunden zur Reaktion gebracht. Anschließend wird über K10-Druckfilter das Ammoniumsalz abgetrennt.
   - Produkt:: SZ: 8 mg KOH/g; JFZ: 10-15; Viskosität: 150 Pas.
7. 350 g Laromer PO 33F wurden mit 175 g Diethanolamin versetzt und bei 50-60°C 4 Stunden zur Reaktion gebracht.
   - Produkt:: SZ: 0,3 mg KOH/g; Viskosität: 4,16 Pas.
   100 g dieses Harzes werden mit 26 g Chlorformiat der Formel III, 14,8 g Triethanolamin und 100 g Essigester vermischt und wie oben beschrieben aufgearbeitet
   - Produkt:: SZ: 33,8 mg KOH/g; Viskosität: 27,5 Pas.

### Anwendungsbeispiele

- A: Das Produkt aus Beispiel 1) wurde auf Papier in einer Schichtdicke von 15 µm aufgetragen und mit einer UV-Lampe (IST, MCX 300 1 Lampe 120 W/cm) bestrahlt. Die Reaktivität betrug 35 m/min, d.h. bei einer Transportgeschwindigkeit von 35 m/min (beschichtete Proben werden unter der UV-Lampe durchgefahren) ergaben sich kratzfeste Überzüge. Die Chemikalienbeständigkeit nach DIN 68 860 B betrug 0,95 (Mittelwert aus 10 Prüfungen)
- Vergleich A: Das in Beispiel 1) verwendete Laromer LR 8812 wurde zum Vergleich mit 4 % Benzophenon versetzt, in einer Schichtdicke von 15 µm auf KD-Papier aufgetragen und mittels UV-Strahlung gehärtet. Die Reaktivität betrug 35 m/min. (Chemikalienbeständigkeit 1,1).
- B: Das Produkt aus Beispiel 2 wurde mit 5 % Wasser verdünnt, mit einer Schichtdicke von 100 µm auf KD-Papier aufgetragen und mit UV-Licht gehärtet. Die Reaktivität betrug 20 m/min. Die Chemikalienbeständigkeit (Mittelwert aus 10 Prüfungen nach DIN 68860 B) ergab eine Note von 1.3 (geringer Zahlenwert ≙ gute Chemikalienbeständigkeit).
- Vergleich B: Das in Beispiel 2) verwendete Butandioldiglycidetherdiacrylat wurde zum Vergleich mit Wasser verdünnt, mit 4 % Iragcure® 500 versetzt, in einer Schichtdicke von 100 µm auf KD-Papier aufgetragen und mit UV gehärtet. Die Reaktivität betrug 30 m/min. Die Chemikalienbeständigkeit (Mittelwert aus 10 Prüfungen nach DIN 68860 B) ergab eine Note von 2.5.

## Patentansprüche

1. Strahlungshärtbare (Meth)acrylate, erhältlich durch Umsetzung von Verbindungen der Formel 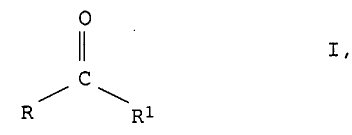 wobei R für einen C₁-C₄-Alkylrest, einen Arylrest oder den Rest R¹ steht und
R¹ für den Rest 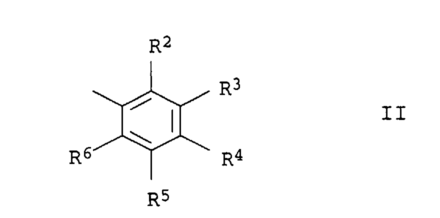 steht, wobei die Reste R² bis R⁶ unabhängig voneinander für H, C₁-C₄-Alkyl, C₁-C₄-Alkoxy, OH, Phenyl, SH, SCH₃, SC₂H₅, F, Cl, Br, CN, COOH, COO-(C₁-C₁₇-Alkyl), COO-(C₅-C₁₀-Aryl), CF₃, N(Alkyl)₂, N((Alkyl)(Aryl), N(Aryl)₂, N^{⊕}(Alkyl)₃ A^{⊖}, N^{⊕} H(Alkyl)₂A^{⊖} stehen, A^{⊖} das Anion einer Säure und Alkyl-, bzw. Aryl-, soweit nicht anderes angegeben, eine C₁-C₁₀-Alkylgruppe bzw. eine C₅-C₁₀-Arylgruppe bedeutet und mindestens einer, aber maximal 3 der Reste R² bis R⁶ eine Gruppe 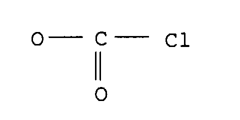 sind, mit Hydroxy(meth)acrylaten, welche mindestens 1 freie Hydroxygruppe und mindestens 2 (Meth)acrylgruppen im Molekül enthalten, bei 0 bis 100°C unter Feuchtigkeitsausschluß.

2. Strahlungshärtbare (Meth)acrylate gemäß Anspruch 1, wobei einer der Reste R² bis R⁶ für eine Gruppe 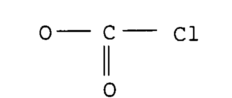 steht.

3. Strahlungshärtbare (Meth)acrylate gemäß Anspruch 1, wobei es sich bei den Hydroxy(meth)acrylaten um Polyether- oder Polyester(meth)acrylate handelt, welche mindestens eine freie Hydroxygruppe und zwei bis sechs Acrylgruppen im Molekül enthalten.

4. Strahlungshärtbare (Meth)acrylate gem. Anspruch 1, wobei es sich bei den Hydroxy(meth)acrylaten um Polyether- oder Polyester(meth)acrylate handelt, bei denen die freie Hydroxygruppe über Michaeladdukten der (Meth)acrylgruppen mit Alkanolaminen ins Harz eingebaut wurde.

5. Verfahren zur Herstellung von stahlungshärtbaren (Meth)acrylaten, dadurch gekennzeichnet, daß Verbindungen der Formel I mit Hydroxy(meth)acrylaten, welche mindestens 1 freie Hydroxygruppe und mindestens 2 (Meth)-Acrylgruppen enthalten, bei 0 bis 100°C unter Feuchtigkeitsausschluß umgesetzt werden.

6. Verwendung von strahlungshärtbaren (Meth)acrylaten gemäß einem der Ansprüche 1 bis 4 in strahlungshärtbaren Massen.

## Claims

1. A radiation-curable (meth)acrylate obtainable by reacting a compound of the formula 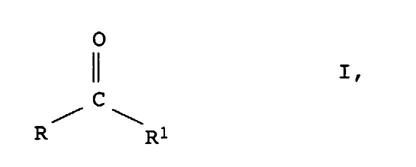 where R is C₁-C₄-alkyl, aryl or R¹ and R¹ is 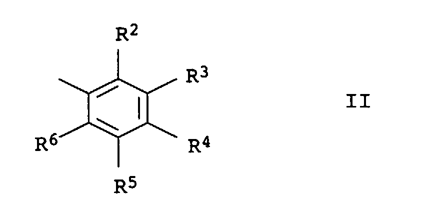 where R² to R⁶ independently of one another are H, C₁-C₄-alkyl, C₁-C₄-alkoxy, OH, phenyl, SH, SCH₃, SC₂H₅, F, Cl, Br, CN, COOH, COO-(C₁-C₁₇-alkyl), COO-C₅-C₁₀-aryl), CF₃, N(alkyl)₂, N(alkyl)(aryl), N(aryl)₂, N^{⊕}(alkyl)₃ A^{⊖}, N^{⊕}H(alkyl)₂A^{⊖}, A^{⊖} is the anion of an acid, and alkyl or aryl, unless indicated otherwise, is C₁-C₁₀-aikyi or C₅-C₁₀-aryl, respectively, and at least one but not more than 3 of R² to R⁶ are 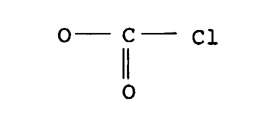 with a hydroxy(meth)acrylate which contains at least 1 free hydroxyl group and at least 2 (meth)acrylic groups in the molecule at from 0 to 100°C with the exclusion of moisture.

2. A radiation-curable (meth)acrylate as claimed in claim 1, wherein one of R² to R⁶ is

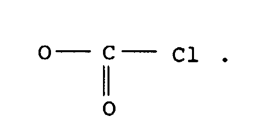

3. A radiation-curable (meth)acrylate as claimed in claim 1, wherein the hydroxy(meth)acrylate is a polyether or polyester (meth)acrylate which contains at least one free hydroxyl group and from two to six acrylic groups in the molecule.

4. A radiation-curable (meth)acrylate as claimed in claim 1, wherein the hydroxy(meth)acrylate is a polyether or polyester (meth)acrylate in which the free hydroxyl group has been incorporated into the resin via Michael adducts of the (meth)acrylic groups with alkanolamines.

5. A process for the preparation of a radiation-curable (meth)acrylate, wherein a compound of the formula I is reacted with hydroxy(meth)acrylates which contain at least one free hydroxyl group and at least 2 (meth)acrylic groups at from 0 to 100°C with the exclusion of moisture.

6. The use of a radiation-curable (meth)acrylate as claimed in any one of claims 1 to 4 in a radiation-curable composition.

## Revendications

1. (Méth)acrylates durcissables par irradiation, pouvant être obtenus par réaction de composés de formule 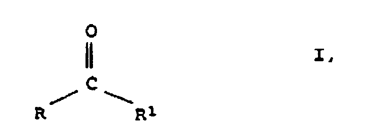 dans laquelle R représente un résidu alkyle en C₁ à C₄, un résidu aryle ou le résidu R¹ et
R¹ représente le résidu 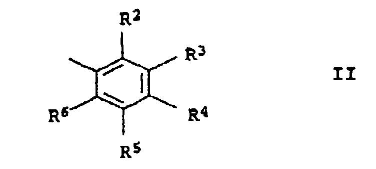 où les résidus R² à R⁶ représentent, indépendamment les uns des autres, un atome de H, un groupe alkyle en C₁ à C₄, alcoxy en C₁ à C₄, OH, phényle, SH, SCH₃, SC₂H₅, F, Cl, Br, CN, COOH, COO-(alkyle en C₁ à C₁₇), COO-(aryle en C₅ à C₁₀),CF₃, N(alkyle)₂, N((alkyle)(aryle)), N(aryle)₂, N^{⊕}(alkyle)₃A^{⊖}, N^{⊕}H(alkyle)₂A^{⊖}, A^{⊖} représente l'anion d'un acide, et un groupe alkyle ou aryle, sauf indication contraire un groupe alkyle en C₁ à C₁₀ ou groupe aryle en C₅ à C₁₀, et au moins un, mais au maximum 3 des résidus R² à R⁶ sont des groupes 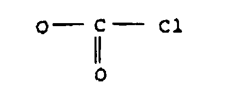 avec des hydroxy(méth)acrylates, qui contiennent au moins un groupe hydroxy libre et au moins 2 groupes (méth)acryles dans la molécule, à une température de 0°C à 100°C, à l'abri de l'humidité.

2. (Méth)acrylates durcissables par irradiation selon la revendication 1, dans lesquels un des résidus R² à R⁶ représente un groupe

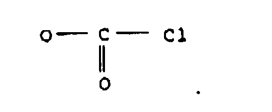

3. (Méth)acrylates durcissables par irradiation selon la revendication 1, dans lesquels, quant aux hydroxy(méth)acrylates, il s'agit de (méth)acrylates de polyester ou de polyéther, qui contiennent au moins un groupe hydroxy libre et deux à six groupes acryles dans la molécule.

4. (Méth)acrylates durcissables par irradiation selon la revendication 1, dans lesquels, quant aux hydroxy(méth)acrylates, il s'agit de (méth)acrylates de polyester ou de polyéther, dans lesquels le groupe hydroxy libre est incorporé dans la résine par des produits d'addition de Michael des groupes (méth)acryles avec des alcanolamines.

5. Procédé de préparation de (méth)acrylates durcissables par irradiation, caractérisé en ce que l'on met à réagir des composés de formule I avec des hydroxy(méth)acrylates, qui contiennent au moins un groupe hydroxy libre et au moins 2 groupes (méth)acryles, à une température de 0°C à 100°C à l'abri de l'humidité.

6. Utilisation de (méth)acrylates durcissables par irradiation selon l'une quelconque des revendications 1 à 4, dans des masses durcissables par irradiation.
